# EUROPEAN PATENT APPLICATION

(11) **EP 3 391 757 A1**
(43) Date of publication of application: **24.10.2018**
(21) Application number: 16876082.5
(22) Date of filing: 16.12.2016
(51) Int. Cl.: A23L 29/00, A23P 10/30, A23L 33/10

(54) **COATING METHOD OF LACTIC ACID BACTERIA WITH INCREASED INTESTINAL SURVIVAL RATE**

(30) Priority: 17.12.2015 KR 20150180965
(71) Applicant: Cj Cheiljedang Corporation, Seoul 04560 (KR)
(72) Inventor: SHIN, Dong Joo, Suwon-si Gyeonggi-do 16508 (KR); YEO, Marie, Yongin-si Gyeonggi-do 16948 (KR); KIM, Tae Hyun, Suwon-si Gyeonggi-do 16516 (KR); MOON, Byoung Seok, Anyang-si Gyeonggi-do 14105 (KR); PARK, Jae Seung, Seoul 08293 (KR); KIM, Sung Ki, Gyeonggi-do 16538 (KR); KIM- Hye Jin, Gyeonggi-do 16505 (KR)
(74) Representative: Cabinet Beau de Loménie
(86) International application number: PCT/KR2016/014824
(87) International publication number: WO 2017/105140

(57) **Abstract**

This application relates to a coating method of lactic acid bacteria and a lactic acid bacteria complex produced by the coating method, the coating method comprising: (a) a step of culturing lactic acid bacteria in a medium including casein and coating the lactic acid bacteria with casein; (b) a step of mixing the casein-coated lactic acid bacteria with a solution comprising a coating agent, an edible oil, an extracellular polymeric substance (EPS) of *Lactobacillus plantarum* and alginic acid; and (c) a step of adding the mixture of step (b) to a calcium-containing solution to form alginic acid-calcium beads, wherein the alginic acid-calcium beads contain the casein-coated lactic acid bacteria, the coating agent, the edible oil, and the EPS of *Lactobacillus plantarum* are contained within the alginic acid-calcium beads.

## Description

### [Technical Field]

The present invention relates to a method for coating a lactic acid bacterium and a lactic acid bacterium complex prepared by the method.

### [Background Art]

Lactic acid bacteria inhabit the intestines of mammals and prevent abnormal fermentation caused by harmful bacteria. Due to this advantage, lactic acid bacteria are important bacteria for intestinal regulation. For example, *L. bulgaricus,* the first known lactic acid bacterial species, is used for yogurt production. *L. bulgaricus* is also used as a starter for the production of cheese and cultured butter. *L. acidophilus* is an aerobic lactic acid bacterium that exists in the intestines of all mammalian animals, including humans, and is used for the production of butter and milk or the treatment of intestinal autointoxication. *L. lactis* produces DL-lactic acid and is used for the production of butter or cheese because it is always found in milk. *L. lactis* is the most important bacterial species for dairy applications.

Such beneficial lactic acid bacteria reside in the intestine and exhibit various physiological activities, such as promotion of intestinal movement, inhibition of harmful bacteria, promotion of vitamin and immunostimulant production, and amelioration of atopic dermatitis. However, such physiological activities are expected only when a much larger amount of lactic acid bacteria than intake of lactic acid bacteria from foods such as yogurt is eaten. Thus, readily edible forms (e.g., powders and capsules) of lactic acid bacterial isolates are currently commercially available.

In many cases, however, lactic acid bacteria processed into the form of powders or capsules are likely to be killed during long-term storage or by gastric acid and bile acid *in vivo.* In order to overcome such disadvantages, recent efforts have been made to develop methods for coating lactic acid bacteria with various coating materials such as starch, gelatin, alginic acid, cellulose, hardened oil and emulsifiers to prepare macrocapsules or microcapsules (≥ 50 µm) and methods for encapsulating lactic acid bacteria to prepare capsules in which functional unsaturated fatty acids are present at higher concentrations than are needed such that the lactic acid bacteria maintain their quality during storage.

### [Disclosure]

### [Technical Problem]

Thus, the present inventors have succeeded in developing a method for coating a lactic acid bacterium by suspension/emulsion and extrusion to achieve markedly improved storage stability and intestinal survival of the lactic acid bacterium, accomplishing the present invention.

### [Technical Solution]

One embodiment of the present invention provides a method for coating a lactic acid bacterium comprising:
(a) culturing a lactic acid bacterium in a medium containing casein to coat the lactic acid bacterium with the casein;
(b) mixing the casein-coated lactic acid bacterium with a solution comprising a coating agent, an edible oil or fat, extracellular polymeric substances (EPSs) of *Lactobacillus plantarum,* and alginic acid; and
(c) adding the mixture to a calcium-containing solution to form calcium alginate beads wherein the casein-coated lactic acid bacterium, the coating agent, the edible oil or fat, and the EPSs of *Lactobacillus plantarum* are containedt within the calcium alginate beads.

A further embodiment of the present invention provides a coated lactic acid bacterium complex prepared by the method. The lactic acid bacterium complex may comprise calcium alginate beads, a casein-coated lactic acid bacterium, EPSs of *Lactobacillus plantarum,* a coating agent, and an edible oil or fat.

Hereinafter, embodiments of the present invention will now be described in detail.

### (a) Culturing lactic acid bacterium in medium containing casein to coat the lactic acid bacterium with the casein

The lactic acid bacterium may be any of those that produce acid and can proliferate even under weakly acidic conditions. The lactic acid bacterium may be selected from, without being limited to, *Lactobacillus sp., Bifidobacterium sp., Streptococcus sp., Lactococcus sp., Enterococcus sp., Pediococcus sp., Leuconostoc sp.,* and *Weissella sp.* Specifically, the lactic acid bacterium may be selected from *Lactobacillus plantarum, Lactobacillus casei, Lactobacillus rhamnosus, Lactobacillus acidophilus, Bifidobacterium bifidum, Bifidobacterium longum, Bifidobacterium breve, Streptococcus faecalis,* and *Lactococcus lactis subsp. lactis.* More specifically, the lactic acid bacterium may be selected from *Lactobacillus plantarum* CJLP243 described in Korean Patent No. 1178217, *Lactobacillus plantarum* CJLP133 described in Korean Patent No. 1486999, *Lactobacillus plantarum* CJLP136 described in Korean Patent No. 1075558, *Lactobacillus plantarum* CJLP55 described in Korean Patent No. 1255050, *Lactobacillus plantarum* CJLP56 described in Korean Patent No. 1075557, and mixtures thereof.

These strains were deposited at the Gene Bank of Korea Research Institute of Bioscience and Biotechnology and are readily available from the Gene Bank of Korea Research Institute of Bioscience and Biotechnology.

The casein-containing medium may be, for example, a medium containing nonfat dry milk. Casein tends to aggregate naturally to form particles when its isoelectric point (pH 4.6) is reached by organic acids as metabolites of growing lactic acid bacteria. When particulated, casein encapsulates the microorganism to form a casein-microorganism matrix. That is, the lactic acid bacterium is coated with casein. The nonfat dry milk comprising casein may be present in an amount of 0.005 wt% to 0.2 wt%, specifically 0.01 wt% to 0.1 wt%, more specifically 0.02 wt% to 0.05 wt%, based on the total weight of the medium. If the content of the nonfat dry milk is higher than 0.2 wt%, the amount of aggregating casein is large, causing poor suspension efficiency after collection of the bacterium. Meanwhile, if the content of the nonfat dry milk is lower than 0.005 wt%, the presence of a small amount of casein makes it difficult to form a casein-bacterium matrix, leading to low coating efficiency. The content of casein in the defatted milk may be in the range of 20 wt% to 30 wt%.

### (b) Mixing of the casein-coated lactic acid bacterium with solution comprising coating agent, edible oil or fat, extracellular polymeric substances (EPSs) of Lactobacillus plantarum and alginic acid

This step is carried out by suspension and/or emulsion. The casein-coated lactic acid bacterium is collected by centrifugation and mixed with a solution including a coating agent, an edible oil or fat, extracellular polymeric substances (EPSs) of *Lactobacillus plantarum,* and alginic acid.

The coating agent is used for the purpose of protecting the lactic acid bacterium from the external environment. Specifically, the coating agent may be selected from porous polymers, proteins, thickening polysaccharides, and mixtures thereof. The coating agent may be used in an amount of 6 wt% to 61 wt%, specifically 10 wt% to 50 wt%, based on the weight of the lactic acid bacterium.

The porous polymer is a base in the form of porous particles and serves to block the introduction of external moisture and air. The porous polymer may be selected from, without being limited to, maltodextrin, chitosan, starch, polyethylene glycol, triacetin, glycerin, and mixtures thereof. Specifically, the porous polymer may include maltodextrin.

The porous polymer may be used in an amount of 5 wt% to 50 wt%, specifically 10 wt% to 30 wt%, more specifically 14 wt% to 16 wt%, based on the weight of the lactic acid bacterium.

The protein serves to fill the pores. The protein may be selected from, without being limited to, defatted milk, whey protein, soybean protein, and mixtures thereof. Specifically, the protein may be defatted milk or whey protein.

The protein may be used in an amount of 1 wt% to 10 wt%, specifically 2 wt% to 8 wt%, more specifically 3 wt% to 7 wt%, based on the weight of the lactic acid bacterium.

The thickening polysaccharide serves to stabilize the porous polymer and the protein when used in combination with the porous polymer and the protein. The thickening polysaccharide also serves to impart stability to the coating agent. Specifically, the thickening polysaccharide may be selected from, without being limited to, gelatin, pectin, guar gum, agar, xanthan gum, gellan gum, and mixtures thereof. Specifically, the thickening polysaccharide may be xanthan gum or gellan gum.

The thickening polysaccharide may be used in an amount of 0.001 wt% to 1 wt%, specifically 0.005 wt% to 0.5 wt%, more specifically 0.008 wt% to 0.1 wt%, based on the weight of the lactic acid bacterium.

The alginic acid may be in the form of an aqueous solution of an alginate, such as sodium alginate. For example, the concentration of the alginate may be from 2 wt% to 4 wt%. The ratio of the weight of the alginic acid-containing solution to the weight of the casein-coated lactic acid bacterium may be from 1:1 to 10:1, specifically 1:1 to 8:1, more specifically 1:1 to 4:1.

The edible oil or fat may be a saturated fat such as coconut oil or palm oil. For example, the edible oil or fat may be in the form of a solid.

The edible oil or fat may be prepared by a method comprising (a) melting a raw edible oil or fat by heating to 90°C to 110°C and (b) cooling the molten edible oil or fat to a temperature range of 40°C to 60°C. More specifically, the edible oil or fat may be prepared by adding water to a raw edible saturated fat, dissolving the raw edible saturated fat by heating to 100°C, and cooling the solution to 50°C. The weight ratio of the edible oil or fat to the lactic acid bacterium may be from 1:0.005 to 1:0.1. The edible oil or fat may further comprise soybean lecithin. In this case, 0.02 wt% of soybean lecithin is added to the solution, water is added thereto, and the mixture is emulsified using a homogenizer to prepare an emulsion. The emulsion may be used in a concentration of about 10 wt%. The emulsion may be mixed with the lactic acid bacterium in a weight ratio of 1:0.05 to 1:1. In this case, the emulsion may be cooled to 50°C before use.

The extracellular polymeric substances (EPSs) of *Lactobacillus plantarum* are viscous polysaccharides and are helpful in improving the intestinal survival and adherence of the lactic acid bacterium.

The *Lactobacillus plantarum* may be, for example, *Lactobacillus plantarum* CJLP243.

The EPSs of *Lactobacillus plantarum* may be prepared by a method comprising (a) adding 1 wt% to 5 wt% of glucose to a culture medium, (b) culturing *Lactobacillus plantarum* at a temperature of 25°C to 40°C in the medium until the concentration of glucose in the medium is reduced to 0.01 wt% or less, and (c) centrifuging the resulting culture and collecting only the supernatant.

The method may further comprise concentrating the supernatant 3 or 4 times after step (c) or mixing the supernatant with a 7- to 10-fold larger amount of dextrin based on the solid content of the culture broth obtained after step (b), drying the mixture, followed by pulverization into a powder. Specifically, MRS broth (Difco) containing 3 wt% of glucose is sterilized, *Lactobacillus plantarum,* more specifically *Lactobacillus plantarum* CJLP243, as the lactic acid bacterium, is cultured at 30°C until the glucose is used up, the culture is centrifuged, and the filtrate is disinfected and concentrated or processed into a powder before use.

The concentration of residual glucose in the EPSs of *Lactobacillus plantarum* may be 0.01 wt% or less and the Brix of the EPSs of *Lactobacillus plantarum* may be in the range of 7 to 8. The EPSs of *Lactobacillus plantarum* are concentrated 3 or 4 times or mixed with a 7- to 10-fold larger amount of dextrin based on the solid weight of the culture broth, and the concentrate or mixture is dried and processed into a powder before use.

The EPSs may be used in an amount of 1 wt% to 50 wt%, based on the weight of the lactic acid bacterium.

When the casein-coated lactic acid bacterium is mixed with the solution containing the coating agent, the edible oil or fat, the extracellular polymeric substances (EPSs) of *Lactobacillus plantarum,* and the alginic acid, the components may be added simultaneously, sequentially or intermittently. Specifically, the casein-coated lactic acid bacterium is first mixed with the coating agent, water is added to the mixture, and the aqueous mixture is mixed with the alginic acid-containing solution. Alternatively, the casein-coated lactic acid bacterium, the coating agent, and the alginic acid-containing solution may be mixed together. Alternatively, the casein-coated lactic acid bacterium may be first mixed with the alginic acid-containing solution and then the coating agent may be added thereto.

Thereafter, the edible oil or fat and the EPSs are added to the aqueous mixture comprising the casein-coated lactic acid bacterium, the coating agent, and the alginic acid-containing solution, followed by suspension and/or emulsification.

A prebiotic may be optionally further added during mixing in step (b). Specifically, a prebiotic may be added when the lactic acid bacterium is mixed with the coating agent. The prebiotic serves as food for the lactic acid bacterium. The prebiotic may be selected from, without being limited to, fructooligosaccharides, galactooligosaccharides, maltitol, lactitol, inulin, and mixtures. Specifically, the prebiotic may be a fructooligosaccharide or inulin.

The prebiotic may be used in an amount of 0.1 wt% to 5 wt%, based on the weight of the lactic acid bacterium.

A cryoprotectant may be optionally further added during mixing in step (b).

The cryoprotectant serves to prevent damage to or killing of the lactic acid bacterium during freeze-drying. For example, the cryoprotectant may be selected from, without being limited to, dextrin, sucrose, glycerol, mannitol, trehalose, and mixtures thereof. Specifically, the cryoprotectant may include trehalose. Another type of cryoprotectant may be optionally further used.

The cryoprotectant may be used in an amount of 5 wt% to 50 wt%, specifically 10 wt% to 30 wt%, more specifically 14 wt% to 16 wt%, based on the weight of the lactic acid bacterium.

The cryoprotectant may be added in any step before freeze-drying. Specifically, the cryoprotectant may be further added when the lactic acid bacterium is mixed with the coating agent. At this time, it is desirable to add the cryoprotectant in such an amount that the ratio of the total weight of the coating agent and the cryoprotectant to the weight of the casein-coated lactic acid bacterium is from 0.1:1 to 5:1.

### (c) Addition of the mixture obtained in step (b) to calcium-containing solution to form calcium alginate beads within which the casein-coated lactic acid bacterium, the coating agent, the edible oil or fat, and the EPSs of Lactobacillus plantarum are contained

This step is carried out by extrusion. When the lactic acid bacterium coated by suspension and/or emulsion and the alginic acid-containing mixture are allowed to react with a calcium-containing solution, the alginic acid is crosslinked with the calcium to form a matrix. As a result, the lactic acid bacterium is encapsulated in beads in the calcium-containing solution. At this time, the coating agent, the EPSs, and the edible oil or fat mixed in step (b) may also be encapsulated in the beads.

The alginic acid-calcium reaction for the formation of bead particles is carried out at a temperature under room temperature (for example, 25°C). Since this reaction involves less physical stress, there is little influence on the survival of the living bacterium. The calcium alginate bead particles formed by extrusion show pH-dependent release. Thus, the calcium alginate bead particles are not decomposed under acidic conditions such as gastric acid and are slowly decomposed in the neutral intestinal environment, greatly contributing to an improvement in the survival of the lactic acid bacterium in the intestine.

Step (c) comprises:
(1) maintaining the mixture obtained in step (b) at 25°C to 35°C;
(2) stirring a 100 mM to 1 M calcium ion solution; and
(3) dropping the mixture obtained in step (b) into the stirred calcium ion solution to prepare a solution of calcium alginate beads.

Step (c) may further comprise (4) storing the solution of calcium alginate beads at a temperature of 4°C to 20°C for 30 minutes to 60 minutes.

In step (c), the mixture obtained by suspension and/or emulsion in step (b) is maintained at a temperature of 25°C to 35°C. The 0.1 M to 1 M calcium ion solution may be, for example, a 100 mM calcium lactate solution. The calcium ion solution is stirred in a glass beaker. The mixture prepared by suspension and/or emulsion in step (b) is added to the calcium ion solution in the bath by extrusion through a 10 mL syringe needle or is sprayed through a micronozzle to directly react with the calcium ion solution. The calcium ion solution is continuously stirred in the bath to prevent the individual reactant particles from aggregating. The mixture filling the 10 mL syringe is directly dropped into the calcium ion solution in the beaker by pressurizing the syringe. As a result of the reaction, beads are formed. After formation of the beads is finished, the bath containing the beads is stored at 4°C to 20°C for an additional 30 minutes to 60 minutes for aging. This aging can increase the degree of compaction of the particles.

In the case where the mixture is extruded through a syringe needle, the beads are collected through a 20 to 100-mesh sieve. In the case where the mixture is sprayed through a micronozzle, the beads are collected through a 100 to 200-mesh sieve. The collected beads are washed twice with distilled water to remove the remaining calcium solution.

### (d) Freeze-drying of the calcium alginate beads comprising the lactic acid bacterium

The method may further comprise (d) freeze-drying the calcium alginate beads formed in step (c). Specifically, the lactic acid bacterium-containing calcium alginate beads formed in step (c) are transferred to and placed on a freeze-drying tray, maintained at a temperature of -40°C to -70°C for 12 hours to 24 hours, and thawed in a freeze-dryer to remove moisture.

A further embodiment of the present invention provides a coated lactic acid bacterium complex prepared by the method.

The lactic acid bacterium complex may comprise calcium alginate beads, a casein-coated lactic acid bacterium, EPSs of *Lactobacillus plantarum,* a coating agent, and an edible oil or fat. The casein-coated lactic acid bacterium and the EPSs of *Lactobacillus plantarum* may be contained within the calcium alginate beads. Alternatively, the lactic acid bacterium complex may comprise calcium alginate beads within which a casein-coated lactic acid bacterium and EPSs of *Lactobacillus plantarum* are contained. In this case, the lactic acid bacterium complex may optionally further comprise an edible oil or fat, a coating agent, a prebiotic, and/or a cryoprotectant. Specifically, the calcium alginate beads may comprise an edible oil or fat and a coating agent. The lactic acid bacterium complex has a structure in which the casein-coated lactic acid bacterium is included in the calcium alginate beads. This inclusion greatly improves the storage stability and intestinal survival of the lactic acid bacterium. The lactic acid bacterium complex may take the form of a solid, specifically a powder, more specifically a freeze-dried powder.

The individual components of the lactic acid bacterium complex are the same as those described in the coating method and a description thereof is omitted to avoid duplication.

### [Advantageous Effects]

The lactic acid bacterium complex of the present invention is prepared based on suspension and/or emulsion and subsequent extrusion and multiply protects a lactic acid bacterium present therein, achieving improved storage stability and intestinal survival of the lactic acid bacterium.

### [Description of Drawings]

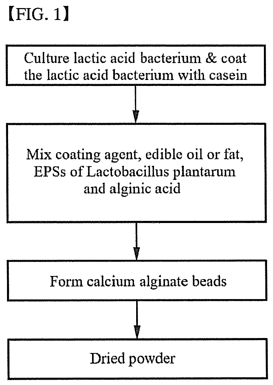

FIG. 1 is a flowchart illustrating a method for preparing a lactic acid bacterium complex according to one embodiment of the present invention.

### [Mode for Invention]

The present invention will be explained in detail with reference to the following examples. However, these examples are merely illustrative and are not to be construed as limiting the scope of the invention.

### Example 1: Preparation of lactic acid bacterium-containing complex

*Lactobacillus plantarum* CJLP243 was cultured in MRS liquid medium (Difco, USA) supplemented with 0.02 wt% of defatted milk at 37°C for 18 to 24 hours. The culture was centrifuged. The supernatant was discarded and only the casein-coated lactic acid bacterium was collected.

Thereafter, about 15 wt% of trehalose as a cryoprotectant, about 15 wt% of maltodextrin as a coating agent, about 4 wt% of defatted milk, about 0.01 wt% of xanthan gum as another coating agent, and 2 wt% of fructooligosaccharide as a prebiotic relative to the weight of the bacterium were mixed together. Water was added to the mixture and sterilized. The collected lactic acid bacterium was mixed with the sterilized solution and the mixture was suspended.

Then, 2 wt% of sodium alginate was dissolved in water to prepare an alginic acid solution. The suspension was mixed with the alginic acid solution in amounts such that the ratio of the weight of the alginic acid solution to the weight of the lactic acid bacterium was 1:4.

Solid saturated fats, including palm oil, were dissolved in water by heating to 100°C. To the solution was added 0.2 wt% of soybean lecithin relative to the weight of the solution. The resulting mixture was emulsified using a homogenizer to prepare an about 10 wt% emulsion. At the final stage of emulsification, the solid saturated fats and the soybean lecithin emulsion were added together with EPSs in a weight ratio of 1:0.5 relative to the weight of the lactic acid bacterium. The EPSs were prepared by the following procedure. First, 3 wt% of glucose was added to MRS broth medium (Difco) and sterilized. *Lactobacillus plantarum* CJLP243 was cultured in the medium at 30°C until the glucose concentration was reduced to ≤ 0.01 wt%. Thereafter, the culture was centrifuged, and the filtrate was disinfected and concentrated or processed into a powder. The concentrate or culture broth was mixed with a 7- to 10-fold larger amount of dextrin based on solid content, dried, and processed into a powder before use. The concentrate or powder of the EPSs was suspended with the lactic acid bacterium in a concentration ratio of 1:0.2. The suspension was maintained at 30°C before coating to maintain its viscosity at an appropriate level. A 100 mM calcium lactate solution was stirred in a glass beaker. A 10 mL syringe was filled with the lactic acid bacterium-containing mixture and the lactic acid bacterium-containing mixture was directly dropped into the calcium lactate solution by pressurizing the syringe. As a result of the reaction, beads were formed. After the formation of beads was finished, the bath containing the beads was cooled to 4°C and stored for 30 min. The beads were collected through a 100-mesh sieve and washed twice with distilled water. The collected particles were transferred to and placed on a freeze-drying tray, maintained under rapid freezing conditions (≤ -40°C) for 12 to 24 hours, and thawed in a freeze-dryer to remove moisture. As a result, a lactic acid bacterium complex was prepared in the form of a dry powder. The lactic acid bacterium complex had a structure in which the lactic acid bacterium was encapsulated in the calcium alginate beads.

### Comparative Example 1: Preparation of freeze-dried lactic acid bacterium

*Lactobacillus plantarum* CJLP243 was cultured in MRS liquid medium (Difco, USA) at 37°C for 18 to 24 hours. The culture was centrifuged. The supernatant was discarded and only the lactic acid bacterium was collected. About 15 wt% of trehalose relative to the weight of the bacterium was dissolved in water and sterilized. The lactic acid bacterium was mixed with the cryoprotectant and the mixture was suspended. The lactic acid bacterium was collected using a centrifuge. The collected lactic acid bacterium was transferred to and placed on a freeze-drying tray, maintained under rapid freezing conditions (≤ -40°C) for 12 to 24 hours, and thawed in a freeze-dryer to remove moisture.

That is, Comparative Example 1 was distinguished from Example 1 in that the steps of culturing in the casein-containing medium, mixing with the coating agents and the prebiotic, mixing with the alginic acid solution, mixing with the edible oil or fat, mixing with the EPSs, and forming calcium alginate beads were omitted.

### Comparative Example 2: Preparation of freeze-dried and casein-coated lactic acid bacterium (including treatment with coating agents)

*Lactobacillus plantarum* CJLP243 was cultured in MRS liquid medium (Difco, USA) supplemented with 0.02 wt% of defatted milk at 37°C for 18 to 24 hours. The culture was centrifuged. The supernatant was discarded and only the casein-coated lactic acid bacterium was collected.

Thereafter, about 15 wt% of trehalose as a cryoprotectant, about 15 wt% of maltodextrin as a coating agent, about 4 wt% of defatted milk, and about 0.01 wt% of xanthan gum as another coating agent relative to the weight of the bacterium were mixed together. The mixture was dissolved in water and sterilized. The collected lactic acid bacterium was mixed with the sterilized solution and the mixture was suspended. The lactic acid bacterium was collected using a centrifuge. The collected lactic acid bacterium was transferred to and placed on a freeze-drying tray, maintained under rapid freezing conditions (≤ -40°C) for about 12 to 24 hours, and thawed in a freeze-dryer to remove moisture.

That is, Comparative Example 2 was distinguished from Example 1 in that the steps of mixing with the prebiotic, mixing with the alginic acid solution, mixing with the edible oil or fat and the EPSs, and forming calcium alginate beads were omitted.

### Comparative Example 3: Preparation of freeze-dried complex in which casein-coated lactic acid bacterium was present in calcium alginate beads (including treatment with coating agents, edible oil or fat, and prebiotic but without treatment with EPSs)

*Lactobacillus plantarum* CJLP243 was cultured in MRS liquid medium (Difco, USA) supplemented with 0.02 wt% of defatted milk at 37°C for 18 to 24 hours. The culture was centrifuged. The supernatant was discarded and only the casein-coated lactic acid bacterium was collected.

Thereafter, about 15 wt% of trehalose as a cryoprotectant, about 15 wt% of maltodextrin as a coating agent, about 4 wt% of defatted milk, about 0.01 wt% of xanthan gum as another coating agent, and 2 wt% of fructooligosaccharide as a prebiotic relative to the weight of the bacterium were mixed together. The mixture was dissolved in water and sterilized.

The collected lactic acid bacterium was mixed with the sterilized solution and the mixture was suspended. Then, 2 wt% of sodium alginate was dissolved in water to prepare an alginic acid solution. The suspension was mixed with the alginic acid solution in amounts such that the ratio of the weight of the alginic acid solution to the weight of the lactic acid bacterium was 1:4. Solid saturated fats, including palm oil, were dissolved in water by heating to 100°C. To the solution was added 0.2 wt% of soybean lecithin relative to the weight of the solution. The resulting mixture was emulsified using a homogenizer to prepare an about 10 wt% emulsion. The emulsion was cooled to 50°C. At the final stage of emulsification, the solid saturated fats and the soybean lecithin were added to the suspension in a weight ratio of 1:0.5 relative to the weight of the lactic acid bacterium. The suspension was maintained at 30°C to maintain its viscosity at an appropriate level. A 100 mM calcium lactate solution was stirred in a glass beaker. A 10 mL syringe was filled with the lactic acid bacterium-containing mixture and the lactic acid bacterium-containing mixture was directly dropped into the calcium lactate solution by pressurizing the syringe. As a result of the reaction, beads were formed. After formation of beads was finished, the bath containing the beads was cooled to 4°C and stored for 30 min. The beads were collected through a 100-mesh sieve and washed twice with distilled water. The collected particles were transferred to and placed on a freeze-drying tray, maintained under rapid freezing conditions (≤ -40°C) for about 12 to 24 hours, and thawed in a freeze-dryer to remove moisture.

That is, Comparative Example 3 was distinguished from Example 1 in that the step of mixing with the EPSs was omitted.

The lactic acid bacterium complexes prepared in Example 1 and Comparative Example 3 and the freeze-dried products of the lactic acid bacterium prepared in Comparative Examples 1 and 2 were evaluated for intestinal survival and storage stability by the following respective procedures. Results are shown in Tables 1 to 4.

### Experimental Example 1: Evaluation of intestinal survival

Lactic acid bacteria are likely to be killed by various environmental factors in the digestive organs after being eaten. The most important factors are gastric acid from the stomach and bile acid from the duodenum. Specifically, gastric acid directly acts on bacteria to induce their death due to its strong acidity. Bile acid is involved in the killing of bacteria due to the presence of various digestive enzymes (mainly lipases) or the stress of osmotic pressure. A simple model method for evaluating the survival of living bacteria against gastric acid/bile acid is the simulated stomach duodenum passage (SSDP) test proposed by M. G. Vizoso Pinto, C. M. A. P. Franz, U. Schillinger, and W. H. Holzapfel in "Lactobacillus spp. with in-vitro prebiotic properties from human faeces and traditional fermented products," International Journal of Food Microbiology, vol. 109, no. 3, pp. 205-214, 2006. According to a major SSDP test, a predetermined concentration of a living bacterium or a powder of a living bacterium was subjected to stationary culture for 1 hour on MRS medium under acidic conditions (pH conditions (pH 3.0) of the stomach after food ingestion) and for an additional 2 hours under bile acid conditions (artificial bile juice, Oxgall/salts), and survival rates of the bacterium were checked every hour. The continuously applied gastric acid-bile acid conditions in the SSDP test are severer for survival of the bacterium than the individual conditions but are more similar to the actual environment of the digestive tract. Particularly, since the dried powders of lactic acid bacterium are deactivated, they are more susceptible to the severe environment of the SSDP test but are considered more suitable as models that are finally eaten.

Each experimental sample was diluted 1:100 with saline buffer, placed in a sterile bag, and homogenized. The sample was continuously diluted with saline buffer depending on the bacterial mass and plated on MRS agar medium (Agar Plate). The plate was collected. After stationary culture at 37°C for 24 hours under aerobic conditions, the bacterial number was counted (initial bacterial number data).

The MRS broth was completely dissolved in distilled water with stirring to a concentration of 55 g/l, adjusted to pH 3.0 with 5 M HCl with stirring, and sterilized (121°C, 15 min), thereby preparing an acidic MRS medium. 50 ml of the acidic MRS medium was plated in a sterile flask, and 1/100 equivalents of the sample was added and dissolved with sufficient shaking. The exact time when the sample was dissolved was checked. The sample was shaken at 80 rpm at 37°C. 1 hour after culture, 1 ml of the sample was continuously diluted with saline buffer and plated on an MRS agar plate. The plate was collected. After stationary culture at 37°C for 24 hours under aerobic conditions, the bacterial number was counted (1 h data).

10 wt% Oxgall solution was prepared by dissolving 10 wt% of Oxgall (Difco) in distilled water. The Oxgall solution was sterilized at 121°C for 15 min. Immediately after sampling, 20 ml of the sterilized Oxgall solution was added to a flask. Subsequently, 85 ml of a buffer for the artificial bile juice was added and sufficiently shaken. The buffer for the artificial bile juice was prepared by dissolving NaHCO₃ (6.4 g/l), KCl (0.239 g/l), and NaCl (1.28 g/l) in distilled water, adjusting the pH of the solution to 7.4 with 5 M HCl, and sterilizing the pH-adjusted solution at 121°C for 15 min. Shake culture was performed at 80 rpm and 37°C. Thereafter, samples were taken every hour for 2 h, continuously diluted with the buffer, and plated on MRS agar plates. The plates were collected. After stationary culture at 37°C for 24 hours under aerobic conditions, the bacterial numbers were counted (2 h and 3 h data).

**[Table 1]**

| Conditions | 0 hr. (initial) | 1 hr. (gastric acid) | 2 hr. (gastric acid + bile acid) | 3 hr. (gastric acid + bile acid) | Decrement (Log CFU/g) |
|---|---|---|---|---|---|
| Comparative Example 1 | 12.23 | 7.97 | 6.67 | 6.9 | 5.33 |
| Comparative Example 2 | 11.02 | 11.01 | 8.76 | 8.69 | 2.33 |
| Comparative Example 3 | 10.60 | 10.48 | 10.16 | 10.21 | 0.39 |
| Example 1 | 10.44 | 10.45 | 10.02 | 10.16 | 0.28 |

**[Table 2]**

| Conditions | 0 hr. (initial) | 1 hr. (gastric acid) | 2 hr. (gastric acid + bile acid) | 3 hr. (gastric acid + bile acid) | Survival (%) |
|---|---|---|---|---|---|
| Comparative Example 1 | 1.70E+12 | 9.33E+07 | 4.68E+06 | 7.94E+06 | 0.0005 |
| Comparative Example 2 | 1.05E+11 | 1.02E+11 | 5.75E+08 | 4.90E+08 | 0.47 |
| Comparative Example 3 | 3.98E+10 | 3.02E+10 | 1.45E+10 | 1.62E+10 | 41 |
| Example 1 | 2.75E+10 | 2.82E+10 | 1.05E+10 | 1.45E+10 | 52 |

Table 1 shows the log values of the experimental results and Table 2 shows the found experimental results. As for Comparative Example 1, the bacterial numbers decreased by about 4.3 log and about 1.1 log in gastric acid and bile acid, respectively. As for Comparative Example 2, the bacterium was stable in gastric acid but its number decreased by about 2.3 log in bile acid. As for Comparative Example 3, the bacterial numbers decreased by about 0.4 log in gastric acid/bile acid. However, the survival of the bacterium in the complex prepared in Comparative Example 3 was improved by about 2 log (∼100 times) compared to that of the freeze-dried bacterium prepared in Comparative Example 2. The survival of the bacterium in the complex prepared in Example 1 was improved by ≥ 0.1 log compared to that of the bacterium in the complex prepared in Comparative Example 3.

### 2. Evaluation of storage stability (at 50°C for 72 h)

Lactic acid bacteria in the form of freeze-dried powders gradually lost their activity depending on storage temperature and period. Generally, factors affecting the activity of lactic acid bacteria include temperature, oxygen, and moisture. Freeze-dried powders of lactic acid bacteria primarily undergo a significant reduction in content at the initial stage of storage due to their very high hygroscopicity. Many methods for improving the storage stability of lactic acid bacteria are known, for example, by applying oxygen absorbers to packaging materials or dehumidifying packaging materials. Ultimately, the storage period of powders of lactic acid bacteria greatly depends on how much they are coated. In attempts to reduce hygroscopicity resulting from the characteristics of raw materials, excipients (e.g., glucose and dextrin) are added in 1 to 10-fold larger amounts than powders before storage. In this experiment, a mixture of maltodextrin and anhydrous crystalline glucose (1:1) as excipients was mixed with the powder in a ratio of 3:1 before storage. To secure air tightness during storage, the samples were individually packaged in aluminum pouches before storage. The packaged samples were analyzed for survival during storage under short-term severe conditions (50°C, 3 days).

A predetermined amount of each of the samples in the form of freeze-dried powders prepared in Comparative Examples 1-3 and Example 1 was packaged and sealed in an aluminum pouch and stored in an incubator at 50°C for 72 hours. The experimental sample was diluted 1:100 with saline buffer, placed in a sterile bag, and homogenized. The sample continuously diluted with saline buffer and plated on an MRS agar plate. The plate was collected. After stationary culture at 37°C for 24 hours under aerobic conditions, the bacterial number was counted.

**Table 3**

| | Comparative Example 1 | Comparative Example 2 | Comparative Example 3 | Example 1 |
|---|---|---|---|---|
| Initial | 10.95 | 10.72 | 10.68 | 10.52 |
| 50°C, 72 hr. | 4.88 | 8.75 | 9.74 | 9.91 |
| Decrement ( Log) | 6.07 | 1.97 | 0.94 | 0.61 |

**Table 4**

| | Comparative Example 1 | Comparative Example 2 | Comparative Example 3 | Example 1 |
|---|---|---|---|---|
| Initial | 8.90E+10 | 5.25E+10 | 4.79E+10 | 3.31E+10 |
| 50°C, 72 hr. | 7.60E+04 | 5.62E+08 | 5.50E+09 | 8.13E+09 |
| Survival (%) | 0.00009 | 1.1 | 11.5 | 24.6 |

The activities of the bacteria before and after storage under short-term severe conditions were measured and compared. Table 3 shows the log values of the experimental results and Table 4 shows the found experimental results. As for Comparative Example 2, a reduction in activity by about 2 log was observed. As for Comparative Example 3, the survival of the bacterium was improved by about 0.9 log. As for Example 1, the survival of the bacterium was further improved to a level of 0.6 log.

## Claims

1. A method for coating a lactic acid bacterium, comprising:
(a) culturing a lactic acid bacterium in a medium containing casein to coat the lactic acid bacterium with the casein;
(b) mixing the casein-coated lactic acid bacterium with a solution comprising a coating agent, an edible oil or fat, extracellular polymeric substances (EPSs) of *Lactobacillus plantarum,* and alginic acid; and
(c) adding the mixture to a calcium-containing solution to form calcium alginate beads,
wherein the calcium alginate beads contain the casein-coated lactic acid bacterium, the coating agent, the edible oil or fat, and the EPSs of *Lactobacillus plantarum.*

2. The method for coating a lactic acid bacterium according to claim 1, wherein the lactic acid bacterium comprises at least one bacterial species selected from the group consisting of *Lactobacillus sp., Bifidobacterium sp., Streptococcus sp., Lactococcus sp., Enterococcus sp., Pediococcus sp., Leuconostoc sp.,* and *Weissella sp.*

3. The method for coating a lactic acid bacterium according to claim 1, wherein the lactic acid bacterium comprises at least one bacterial species selected from the group consisting of *Lactobacillus plantarum, Lactobacillus casei, Lactobacillus rhamnosus, Lactobacillus acidophilus, Bifidobacterium bifidum, Bifidobacterium longum, Bifidobacterium breve, Streptococcus faecalis,* and *Lactococcus lactis subsp. lactis.*

4. The method for coating a lactic acid bacterium according to claim 1, wherein the lactic acid bacterium comprises at least one bacterial species selected from the group consisting of *Lactobacillus plantarum* CJLP243, *Lactobacillus plantarum* CJLP133 *Lactobacillus plantarum* CJLP136, *Lactobacillus plantarum* CJLP55, and *Lactobacillus plantarum* CJLP56.

5. The method for coating a lactic acid bacterium according to claim 1, wherein the casein-containing medium is a medium comprising defatted milk.

6. The method for coating a lactic acid bacterium according to claim 1, wherein the coating agent is selected from the group consisting of porous polymers, proteins, thickening polysaccharides, and mixtures thereof.

7. The method for coating a lactic acid bacterium according to claim 1, wherein the solution used in step (b) further comprises a prebiotic.

8. The method for coating a lactic acid bacterium according to claim 1, wherein the alginic acid is in the form of an aqueous solution of 2 wt% to 4 wt% of sodium alginate and the ratio of the weight of the alginic acid solution to the weight of the casein-coated lactic acid bacterium is from 1:1 to 10:1.

9. The method for coating a lactic acid bacterium according to claim 1, wherein the *Lactobacillus plantarum* is *Lactobacillus plantarum* CJLP243.

10. The method for coating a lactic acid bacterium according to claim 1, further comprising:
freeze-drying the calcium alginate beads containing the casein-coated lactic acid bacterium, the coating agent, the edible oil or fat, and the EPSs of *Lactobacillus plantarum.*

11. The method for coating a lactic acid bacterium according to claim 1, wherein the solution used in step (b) further comprises a cryoprotectant.

12. A lactic acid bacterium complex comprising a casein-coated lactic acid bacterium, a coating agent, an edible oil or fat, EPSs of *Lactobacillus plantarum,* and calcium alginate beads.

13. The lactic acid bacterium complex according to claim 12, wherein the calcium alginate beads contain the casein-coated lactic acid bacterium and the EPSs of *Lactobacillus plantarum.*

14. The lactic acid bacterium complex according to claim 12, further comprising at least one additive selected from the group consisting of prebiotics and cryoprotectants.

15. The lactic acid bacterium complex according to claim 12, wherein the coating agent is selected from the group consisting of porous polymers, proteins, thickening polysaccharides, and mixtures thereof.

16. The lactic acid bacterium complex according to claim 12, wherein the lactic acid bacterium comprises at least one bacterial species selected from the group consisting of *Lactobacillus sp., Bifidobacterium sp., Streptococcus sp., Lactococcus sp., Enterococcus sp., Pediococcus sp., Leuconostoc sp.,* and *Weissella sp.*

17. The lactic acid bacterium complex according to claim 12, wherein the lactic acid bacterium comprises at least one bacterial species selected from the group consisting of *Lactobacillus plantarum, Lactobacillus casei, Lactobacillus rhamnosus, Lactobacillus acidophilus, Bifidobacterium bifidum, Bifidobacterium longum, Bifidobacterium breve, Streptococcus faecalis,* and *Lactococcus lactis subsp. lactis.*

18. The lactic acid bacterium complex according to claim 12, wherein the lactic acid bacterium comprises at least one bacterial species selected from the group consisting of *Lactobacillus plantarum* CJLP243, *Lactobacillus plantarum* CJLP133 *Lactobacillus plantarum* CJLP136, *Lactobacillus plantarum* CJLP55, and *Lactobacillus plantarum* CJLP56.

19. The lactic acid bacterium complex according to claim 12, wherein the *Lactobacillus plantarum* is *Lactobacillus plantarum* CJLP243.
